(19) Europäisches Patentamt
European Patent Office
Office européen des brevets

(11) **EP 4 089 687 A1**

(12) **EUROPEAN PATENT APPLICATION**

(43) Date of publication:
**16.11.2022 Bulletin 2022/46**

(21) Application number: **21173588.1**

(22) Date of filing: **12.05.2021**

(51) International Patent Classification (IPC):
*G16H 50/20* (2018.01)   *G16H 50/30* (2018.01)
*A61B 5/00* (2006.01)   *G16H 10/40* (2018.01)
*G16H 10/60* (2018.01)   *A61B 5/346* (2021.01)
*G06N 3/08* (2006.01)

(52) Cooperative Patent Classification (CPC):
**G16H 50/20; A61B 5/412; A61B 5/4842;
A61B 5/7267; G16H 50/30;** A61B 5/346;
G06N 3/08; G16H 10/40; G16H 10/60

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventors:
• **GUPTA, Ankit
560100 Bangalore (IN)**

• **DIETRICH, Carsten
90429 Nürnberg (DE)**
• **G, Saravanan
600029 Chennai, Tamil Nadu (IN)**
• **VALLINES, Lisa
91085 Weisendorf (DE)**

(74) Representative: **Horn Kleimann Waitzhofer
Patentanwälte PartG mbB
Ganghoferstraße 29a
80339 München (DE)**

(54) **METHOD AND A DEVICE FOR DETERMINING ONSET OF SEPSIS**

(57)     A method (200), and a device (100) for determining onset of sepsis is provided. In one aspect, the method (200) includes receiving at least one medical dataset associated with the patient, wherein the medical dataset comprises a plurality of features. Further, the method (200) includes extracting one or more features from the medical dataset, wherein the one or more features comprises parameters associated with the patient which are indicators of sepsis. Additionally, the method (200) includes imputing at least one missing value in the medical dataset, wherein the missing value is associated with the features in the medical dataset. The method (200) also includes determining an output parameter indicative of the onset of sepsis in the patient by using the one or more features and the at least one missing value in the medical dataset as an input for one or more trained machine learning model (700). Furthermore, the method (200) includes generating an alert (ALT) indicating the onset of sepsis in the patient if the output parameter fulfills a predefined criterion associated with sepsis.

FIG 2

**Description**

The present invention relates to a method and a device for determining an onset of sepsis in a patient.

Sepsis is an extreme immune response of an individual's body to an infection. Sepsis can be life-threatening, triggering a series of reactions throughout the body which may cause tissue damage, organ failure and death. Early detection of sepsis is considered as one of the key aspects to improve the outcomes of sepsis treatments. Clinical criteria that assist in sepsis recognition are widely available, however the fundamental need for early detection and treatment of sepsis remains unmet. Existing methods of early detection of sepsis do not achieve levels of specificity and sensitivity required for accurate detection of sepsis in hospital settings. Additionally, existing methods of early detection of sepsis are marred by limitations of imbalanced medical datasets and skewed results.

Currently, there is no way in which the onset of sepsis in a patient can be detected early on with high specificity. Therefore, there is a need for a method and device which enables timely determination of onset of sepsis in a patient, that is effective and accurate.

The object of the invention is therefore to provide a method and a device that enables effective determination of onset of sepsis in a patient at an early time stage.

The invention achieves the object by a method of determining onset of sepsis in a patient. The method comprises receiving at least one medical dataset associated with the patient. The medical dataset includes, for example, medical information associated with the patient. The medical information may include a plurality of features such as, but not limited to, laboratory parameters obtained from a blood sample analysis (such as blood analyte measurements), vital statistics such as heart rate, etc. and patient demographic data such as age of the patient, gender of the patient, etc. In an embodiment, the medical dataset may be stored in a medical database and may be accessed when needed. The method comprises extracting one or more features from the medical dataset, wherein the one or more features include parameters associated with the patient which are indicators of sepsis. In an embodiment, the medical dataset may additionally include features which may not be indicative of sepsis. Therefore, the one or more features in the medical dataset which may support determination of onset of sepsis in the patient are extracted for further processing. The method further comprises imputing at least one missing value in the medical dataset. For example, the missing value may be associated with the features in the medical dataset. For example, the heart rate associated with the patient may not have been recorded for a time period X. This may create a missing value for the heart rate in the medical dataset, for the time period X. Imputation of such missing value is performed to ensure that the medical dataset is complete.

The method further comprises determining an output parameter indicative of the onset of sepsis in the patient by using the one or more features as an input for a trained machine learning model. The trained machine learning model may be configured to process the one or more features in the medical dataset and generate the output parameter indicative of the onset of sepsis in the patient. For example, the output parameter may indicate an onset of sepsis in the patient or no presence of sepsis in the patient. In an embodiment, the trained machine learning model may be a recurrent neural network with long-short term memory. The method further comprises generating an alert indicating the onset of sepsis in the patient. The alert may be generated, for example, if the output parameter generated by the trained machine learning model fulfils a pre-defined criterion associated with sepsis. In an embodiment, the alert may be generated on an output device used by a user. The alert may be, for example, a notification indicating the onset of sepsis in the patient. The notification may be generated on a graphical user interface of the output device. Alternatively, the notification may also include a sound and/or a haptic feedback provided on the output device. Advantageously, the method enables early identification of onset of sepsis in the patient. Therefore, the patient can be treated effectively and in a timely manner.

According to an embodiment, the method further comprises normalizing values associated with the one or more features in the medical dataset which are provided as an input to the trained machine learning model. In an embodiment, normalizing the values of the one or more features includes defining a uniform minimum and maximum threshold for each of the one or more features in the medical dataset. Generally, each feature in the medical dataset may have a varied minimum and maximum threshold value. Normalization enables bringing all the threshold values to a same level such that effective analysis of the dataset is made possible for a trained machine learning model. In an embodiment, the minimum and maximum threshold values of the features are redefined to a range of minimum value of 1 and a maximum value of 5. If the values of the one or more features associated with the patient lie outside the range of 1 and 5, the values may be replaced by minimum and maximum threshold values associated with the features. It is to be noted that use of large values for normalization of the threshold values may cause a loss of temporal changes in the features, over a period of time. Therefore, the threshold values may be capped at minimum value of 1 and maximum value of 5. Advantageously, the invention enables capturing even the smallest change in the values of the one or more features associated with the patient. This enables achieving a high specificity in determination of the onset of sepsis.

According to another embodiment, imputing the at least one missing value in the medical dataset comprises identifying a missing value associated with the features in the medical dataset. In an embodiment, a value associated with one or more features in the medical dataset may be missing if the values may not have been captured/recorded for the patient, at a given time interval. Alternatively, the value associated with the one or more features in the medical dataset may be considered as missing if the values are un-plausible or beyond reasonable thresholds. The method further comprises determining a value preceding the missing value associated with the features. The value preceding the missing value may be the value last captured/recorded for the patient before the given time interval. The method further comprises substituting the missing value with the value preceding the missing value associated with the features in the medical dataset. In an embodiment, the substitution of the missing value with the preceding value may be performed for a limited time period within which the values are recorded. The time period may be dependent on a type of the one or more features in the medical dataset. For example, the time period defined for vital signs may be limited at a range of 4-6 hours and for laboratory parameters at a range of 22 to 24 hours. Advantageously, the medical dataset is made complete and more usable by the trained machine learning model. Therefore, the accuracy of determination of the onset of sepsis in the patient is improved.

According to an embodiment, the one or more features in the medical dataset comprises at least one of vital signs associated with the patient, analytes present in a blood sample of the patient, demographic data associated with the patient, and derivative parameters associated with the vital signs and the analytes present in a blood sample of the patient. For example, the vital signs associated with the patient include, but are not limited to, heart rate associated with the patient, pulse oximetry measurements, body temperature of the patient, systolic blood pressure, mean arterial pressure, diastolic blood pressure, and respiration rate. For example, analytes present in the blood sample associated with the patient include, but are not limited to, blood urea nitrogen, creatinine, lactate, bilirubin, white blood cells, platelets, pH of the blood and serum glucose. Patient demographic data includes, for example, age and gender associated with the patient. Derivative features associated with the patient include, but are not limited to, shock index associated with the patient, ratio of blood urea nitrogen to creatinine, Modified Early Warning Score (MEWS) and partial SOFA (acronym for Sequential Organ Failure Assessment) score.

According to another embodiment, the output parameter indicative of the onset of sepsis in the patient is a probability value generated by the trained machine learning model. The machine learning model may be configured to process the one or more features and generate a probability value associated with the onset of sepsis in the patient. The advantage of the invention is that the probability value is based on the one or more features associated with the patient. Therefore, any change in the values associated with the one or more features will cause the probability value to also change, thereby providing an accurate measure of the onset of sepsis in the patient.

According to an embodiment, generating the alert indicative of the onset of sepsis in the patient comprises determining if the probability value associated with the patient exceeds a first pre-defined threshold. The first pre-defined threshold may be a threshold associated with the probability value generated by the machine learning model. If the probability value exceeds the first pre-defined threshold, a warning or alert may be generated. For example, the first pre-defined threshold may be in the range of 0.4 to 1.0, preferably between 0.5 to 1.0. In an embodiment, the first pre-defined threshold may be modified according to a preference of the user associated with the patient. In another embodiment, the warning may be generated if the probability value falls within the first pre-defined threshold. Advantageously, the user (such as a physician or a medical personnel) is alerted promptly if the probability of the onset of sepsis in the patient is identified or increases. Therefore, necessary medical action may be taken in a timely manner to save the life of the patient.

According to yet another embodiment, generating the alert further comprises identifying a number of generated warnings indicative of the onset of sepsis. For example, the number of warnings generated may be identified for a defined time period for which the patient may be monitored. Further, a determination is made if the number of the generated warnings exceeds a second pre-defined threshold. If the number of generated warnings exceeds the second pre-defined threshold, an alarm is generated indicative of the onset of sepsis in the patient. For example, the second pre-defined threshold may be dependent on the time period for which the patient is monitored. For example, for a defined time period of two hours, the second pre-defined threshold may be two warnings. Therefore, if the number of warnings generated exceeds 2 in a time period of two hours, an alarm may be generated indicative of the onset of sepsis. In an embodiment, the alarm generated may include a notification indicative of the number of warnings/alerts generated for the patient. The alarm may be associated with a sound based or a haptic based output, along with the notification. The advantage of the invention is that the alarm enables the user to identify the progression of the disease in the patient's body early on. Therefore, necessary measures may be implemented to improve the patient's condition in a timely manner.

According to a preferred embodiment, the trained machine learning model is a recurrent neural network, particularly comprising a long-short term memory block. In particular, a recurrent neural network is an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network). In particular, the recurrent neural network can comprise additional storage states or additional network structures that incorporate time delays or comprise feedback loops.

Equivalently, a recurrent neural network could also be de-fined as a neural network whose output does not only depend on the input value and the edge weights, but also on a hidden state vector, wherein the hidden state vector is based on previous inputs used on the recurrent neural network. According to a further aspect of the invention the recurrent neural network comprises at least one long short-term memory (LSTM) block. In particular, an LSTM block comprises a cell, an input gate, an output gate and a forget gate, wherein the cell corresponds to the hidden vector, and the input gate, the output gate and the forget gate regulate the flow of information into and out of the cell. In particular, by using a cell, LSTM blocks can prevent exploding and vanishing gradient problems that can be encountered when training other types of recurrent neural networks.

Advantageously, recurrent neural networks enable temporal analysis of data thereby considering not just a real-time input dataset but also datasets which may have been analyzed previously in time by the neural network. Additionally, LSTM blocks are suited for input data that is separated by diverse or even unknown time intervals.

The object of the invention is also achieved by a method of training a machine learning model for determining an onset of sepsis in a patient. The method comprises receiving a medical dataset associated with the patient, wherein the medical dataset comprises a plurality of features associated with the patient. The medical dataset may be received from a source such as medical database. Additionally, the method comprises extracting one or more features from the plurality of features in the medical dataset, wherein the one or more features comprises parameters associated with the patient which are indicators of onset of sepsis at a given point in time. The one or more features may reflect a medical condition of the patient in real-time or near real-time. Therefore, the one or more features in the medical dataset may indicate the onset of sepsis at a given point in time. Additionally, the method comprises receiving the machine learning model and determining by the model a probability value for the onset of sepsis in the patient based on the one or more features in the medical dataset. In an embodiment, the probability value may be determined based on values associated with the one or more features in the medical dataset.

The method further comprises receiving sepsis data related to the medical dataset, wherein the sepsis dataset indicates an onset of sepsis or indicate no presence of sepsis at a defined time period in the patient associated with the medical dataset. In an embodiment, the sepsis dataset may include a medical dataset which has been labelled to be indicative of onset of sepsis or no presence of sepsis for a defined time period in a patient. In a further embodiment, the labelled medical dataset may be associated with a plurality of patients historically monitored and treated for sepsis. The labelled medical dataset may include one or more features recorded at regular time intervals, thereby showcasing variation in the values associated with the one or more features over the time intervals. In an alternate embodiment, the sepsis data may be data received from a physician/expert which may include an analysis of one or more features present in the medical dataset associated with the patient, indicating the onset of sepsis or presence of no sepsis.

The method further comprises adjusting the machine learning model based on an outcome of comparison between the probability value and the sepsis data. The comparison may indicate an accuracy of the probability value generated by the machine learning model. Therefore, the machine learning model may be adjusted if a difference between the probability value and the sepsis data is identified in the comparison. Advantageously, the machine learning model is made more robust, thereby improving the accuracy with which the probability values are generated by the model. Therefore, determination of the onset of sepsis in the patient may be made effective and timely.

According to an embodiment, the method further comprises pre-processing the sepsis data. The pre-processing may further comprise imputing at least one missing value in the sepsis data. The sepsis data includes the one or more feature values captured/recorded at regular time intervals. In cases where a feature value is missing in the dataset, such value is imputed based on a value preceding in the medical dataset. In an embodiment, a threshold may be defined for such imputation, based on a nature of the feature whose value is missing. For example, if the missing value is associated with vital signs of the patient, the imputation of the missing value can be performed using a value in a range of 4 to 6 hours preceding the missing value. Similarly, if the missing value is associated with the analytes in the blood sample, the imputation of the missing value can be performed using a value in a range of 22 to 24 hours preceding or succeeding the missing value. Advantageously, imputation of values in the sepsis data enables the data to be more complete and useful.

The pre-processing of the sepsis data further comprises normalizing the one or more features from the sepsis data, wherein normalizing comprises defining a uniform minimum and maximum threshold for each of the one or more features. Advantageously, normalization enables bringing all the threshold values to a same level such that effective analysis of the dataset is made possible for the machine learning model. This enables achieving a high specificity in determination of the onset of sepsis.

According to yet another embodiment of the invention, the label associated with the sepsis data is modified such that the defined time period indicated by the label is advanced by a range of 2 to 10 hours, preferably by 3 to 9 hours, more preferably by 6 to 8 hours and most preferably by 5 to 7 hours. Advantageously, modifying the defined time period provides a lookahead time of approximately 5 to 7 hours. Therefore, the machine learning model may be trained in a manner to determine the onset of sepsis in the patient well in advance.

The object of the invention is also achieved by a sepsis determination device for determining an onset of sepsis in a patient. The device comprises one or more processing units, a medical database coupled to the one or more processing units, the medical database comprising a plurality of medical datasets associated with the patient and sepsis data. The device further comprises a memory coupled to the one or more processing units. The memory comprises a sepsis determination module configured to perform the method steps as described above, using at least one trained machine learning model.

The invention relates in one aspect to a computer program product comprising a computer program, the computer program being loadable into a storage unit of a system, including program code sections to make the system execute the method according to an aspect of the invention when the computer program is executed in the system.

The invention relates in one aspect to a computer-readable medium, on which program code sections of a computer program are saved, the program code sections being loadable into and/or executable in a system to make the system execute the method according to an aspect of the invention when the program code sections are executed in the system.

The realization of the invention by a computer program product and/or a computer-readable medium has the advantage that already existing management systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The present invention is further described hereinafter with reference to illustrated embodiments shown in the accompanying drawings, in which:

FIG 1 illustrates a block diagram of a sepsis determination device in which an embodiment for determining onset of sepsis in a patient can be implemented.

FIG 2 illustrates a flowchart of a method of determining the onset of sepsis in a patient, according to an embodiment of the invention.

FIG 3 illustrates a flowchart of a method of imputing missing values in the medical dataset, according to an embodiment of the invention.

FIG 4 illustrates a flowchart of a method of generating an alert indicating the onset of sepsis in the patient, according to an embodiment of the invention.

FIG 5 illustrates a flowchart of a method of training a machine learning model for determining the onset of sepsis in the patient, according to an embodiment of the invention.

FIG 6 illustrates a flowchart of a method of pre-processing sepsis data, according to an embodiment of the invention.

FIG 7 illustrates a working of the machine learning model for determining the onset of sepsis in the patient, according to an embodiment of the invention.

FIG 8 illustrates a graphical representation for monitoring probability value based on which alerts and alarms indicating onset of sepsis in a patient is generated, according to an embodiment of the present invention.

FIG 9 illustrates yet another embodiment of working of the machine learning model for determining the onset of sepsis in the patient, according to an embodiment of the invention.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

In the following, the solution according to the invention is described with respect to the claimed providing systems as well as with respect to the claimed methods. Features, advantages or alternative embodiments herein can be assigned to the other claimed objects and vice versa. In other words, claims for the providing systems can be improved with features described or claimed in the context of the methods. In this case, the functional features of the method are embodied by objective units of the providing system.

Furthermore, in the following the solution according to the invention is described with respect to methods and systems for determining an onset of sepsis in a patient as well as with respect to methods and systems for training a machine learning model for determining an onset of sepsis in a patient. Features, advantages or alternative embodiments herein can be assigned to the other claimed objects and vice versa. In other words, claims for methods and systems for training the machine learning model for determining an onset of sepsis in a patient can be improved with features described or claimed in context of the methods and systems for determining an onset of sepsis in a patient, and vice versa. In particular, the trained machine learning model of the methods and systems for determining an onset of sepsis in a patient can be adapted by the methods and systems for training the machine learning model for determining an onset of sepsis in a patient. Furthermore, the input data can comprise advantageous features and embodiments of the training input data, and vice versa. Furthermore, the output data can comprise advantageous features and embodiments of the output training data, and vice versa.

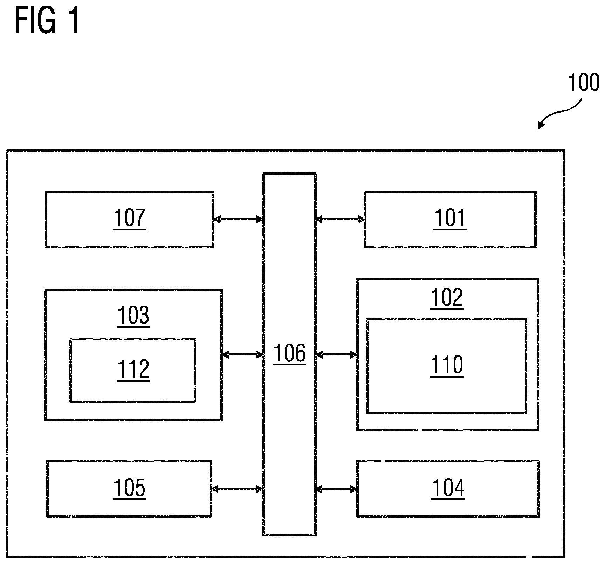

FIG 1 is a block diagram of a sepsis determination device 100 in which an embodiment can be implemented, for example, as a device 100 for determining an onset of sepsis in a patient, configured to perform the processes as described therein. In FIG 1, said device 100 comprises a processing unit 101, a memory 102, a storage unit 103, an input unit 104, a bus 106, an output unit 105, and a network interface 107.

The processing unit 101, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

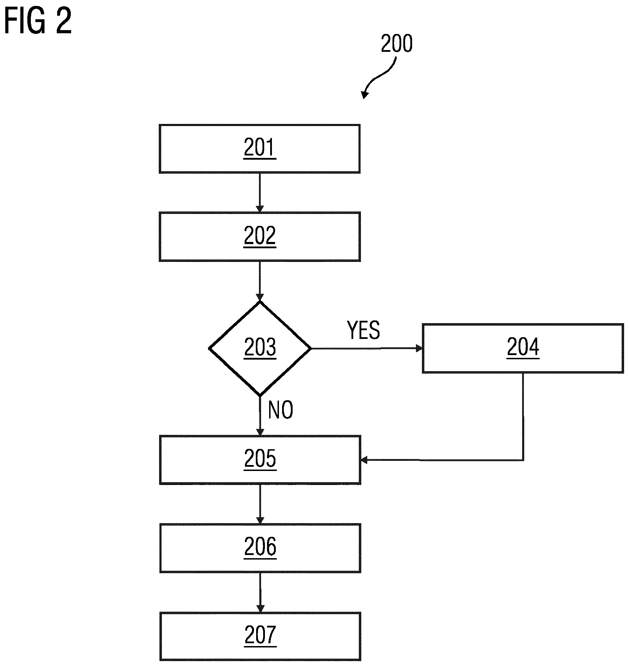

The memory 102 may be volatile memory and non-volatile memory. The memory 102 may be coupled for communication with said processing unit 101. The processing unit 101 may execute instructions and/or code stored in the memory 102. A variety of computer-readable storage media may be stored in and accessed from said memory 102. The memory 102 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 102 includes a sepsis determination module 110 stored in the form of machine-readable instructions on any of said above-mentioned storage media and may be in communication to and executed by processor 101. When executed by the processor 101, the sepsis determination module 110 causes the processor 101 to process a medical dataset to determine an onset of sepsis in a patient. Method steps executed by the processor 101 to achieve the abovementioned functionality are elaborated upon in detail in FIG 2, 3, 4, 5 and 6.

The storage unit 103 may be a non-transitory storage medium which stores a medical database 112. The medical database 112 is a repository of medical dataset and sepsis data related to one or more patients that is maintained by a healthcare service provider. The input unit 104 may include input means such as keypad, touch-sensitive display, camera (such as a camera receiving gesture-based inputs), etc. capable of receiving input signal such as a medical image. The bus 106 acts as interconnect between the processor 101, the memory 102, the storage unit 103, the input unit 104, the output unit 105 and the network interface 107.

Those of ordinary skilled in the art will appreciate that said hardware depicted in FIG 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A device 100 in accordance with an embodiment of the present disclosure includes an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. Said operating system is modified or created in accordance with the present disclosure as described.

Disclosed embodiments provide systems and methods for processing medical dataset. In particular, the systems and methods may enable determination of an onset of sepsis in a patient.

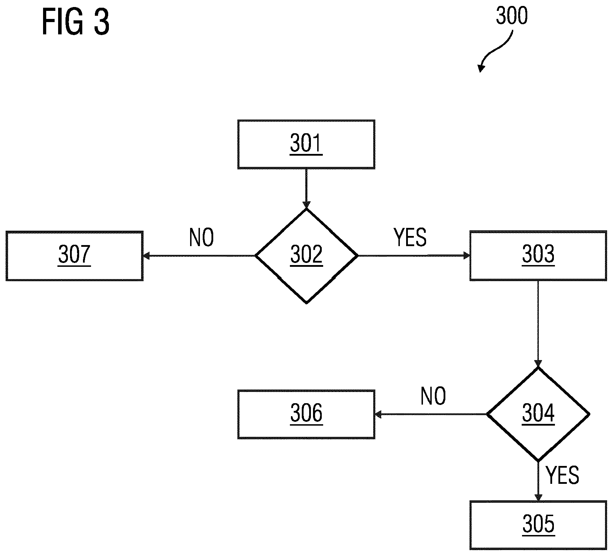

FIG 2 illustrates a flowchart of a method 200 of determining an onset of sepsis in a patient, according to an embodiment of the present invention. At step 201, medical dataset associated with the patient is received from a source. In the present embodiment, the source is the medical database 112. The medical dataset includes a plurality of features associated with the patient. The features relate to medical information such as vital signs associated with the patient, blood analyte information and/or patient demographic data. The features provide inputs essential for determination of onset of sepsis in the patient. In the embodiment, values associated with the features have been captured/recorded at regular time intervals. This enables identification of progression of the patient's condition at regular intervals. Further, at step 202, one or more features are extracted from the plurality of features in the medical dataset. In an embodiment, the medical dataset may include features which may not be essential for determination of onset of sepsis. Therefore, the one or more features which reflect an onset of sepsis in the patient are extracted. Such one or more features include parameters such as, but not limited to, vital signs associated with the patient, laboratory parameters such as blood analyte levels in the patient, patient demographic data, and a plurality of derivative features. The derivative features are derived from the vital signs and the blood analyte levels associated with the patient. Since the values associated with the one or more features are captured at regular time intervals, a check is performed at step 203 to determine a presence of missing values in the medical dataset. If values associated with the extracted one or more features are missing, at step 204, an imputation of the missing values is performed such that the missing value associated with the one or more features is replaced/replicated in the medical dataset. The method steps associated with imputation of the one or more features is described in detail in FIG 3.

At step 205, the one or more features from the medical dataset are normalized. Normalization is an imperative process that is required to be performed before the medical dataset is processed by a trained machine learning model. The values associated with the one or more features may have varied minimum and maximum thresholds. This may result in loss of temporal changes in the dataset. Therefore, normalization of the values associated with the one or more features enables capturing minute temporal changes that the medical dataset may reflect. Therefore, advantageously, any change in the patient's health can be monitored and treated effectively. For example, the below table depicts clinically relevant minimum and maximum thresholds associated with the one or more features in the medical dataset, measured over time.

| Parameter | Minimum | Maximum |
|---|---|---|
| Heart rate (bpm) | 20 | 200 |
| Pulse oximetry (%) | 70 | 100 |
| Temperature (Celsius) | 32 | 42.2 |
| Systolic blood pressure (mmHg) | 40 | 240 |
| Respiratory rate (bpm) | 1 | 50 |
| Diastolic blood pressure (mmHg) | 20 | 150 |
| Mean arterial pressure (mmHg) | 20 | 200 |
| Blood urea nitrogen (mg/dL) | 1 | 120 |
| Creatinine (mg/dL) | 0.1 | 10 |
| Lactate (mg/dL) | 0.2 | 12 |
| Bilirubin total (mg/dL) | 0.1 | 15 |
| White blood cells (10^3/$\mu$L) | 0.1 | 40 |

| Platelets (10^3/$\mu$L) | 1 | 650 |
|---|---|---|
| pH | 6.6 | 7.8 |
| Glucose (mg/dL) | 10 | 400 |

At step 205, the minimum and maximum threshold associated with the one or more feature values is redefined such that the values lie in a range of 1 to 5. The normalization of the one or more feature values is performed using the below mathematical expression:

$$y = \frac{4(x - x_{\min})}{x_{\max} - x_{\min}} + 1$$

where 'y' represents the normalized value associated with the feature, 'x' represents a recorded value of the feature associated with the patient, '$x_{min}$' represents a clinically relevant minimum value associated with the feature and '$x_{max}$' represents a clinically relevant maximum value associated with the feature. In an embodiment, if the value of the feature (x) is missing, 'y' is assigned a value of 0.

In an embodiment, if value of a parameter associated with the patient lies outside the range of 1 and 5, the value is replaced by the minimum or maximum value in the range, respectively.

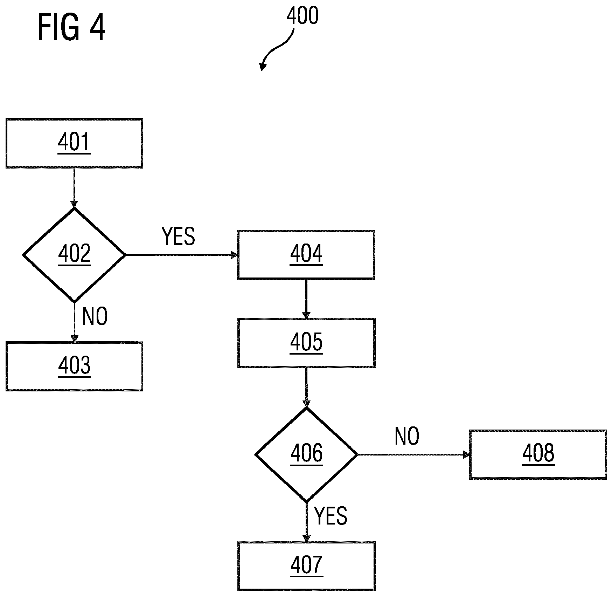

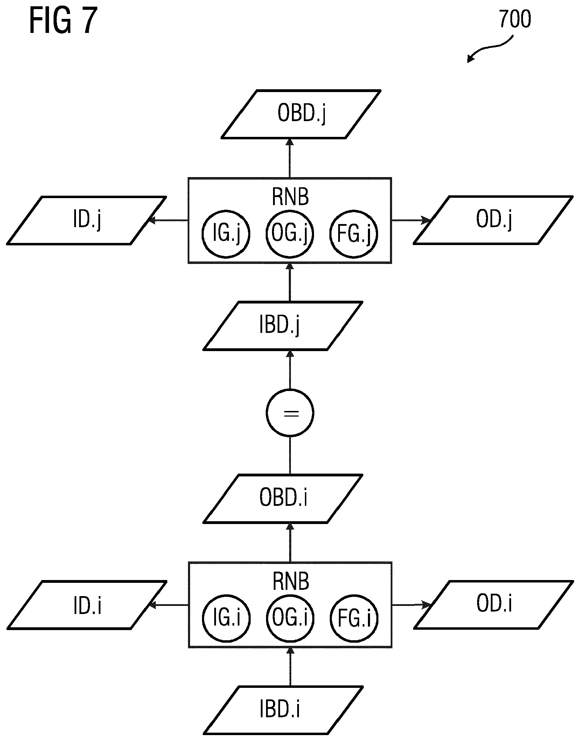

At step 206, an output parameter indicative of the onset of sepsis is determined using the one or more feature values and the imputed values in the medical dataset. The output parameter may be determined by a trained machine learning model. In the present embodiment, the model is a recurrent neural network comprising a long-short term memory block. The working of the machine learning model is described in further detail in FIG 7. The model is configured to process the one or more feature values to generate the output parameter. In the embodiment, the output parameter is a probability value associated with the onset of sepsis in the patient. At step 207, an alert is generated indicative of onset of sepsis in the patient if the output parameter fulfils a pre-defined criterion associated with sepsis. For example, the probability value generated by the model is 0, there is no onset of sepsis in the patient; if the probability value is 1, there is a high chance of an onset of sepsis in the patient. Alternatively, the probability value of 1 may also indicate that the patient has sepsis. In an embodiment, the pre-defined criterion can be defined as per a requirement of a user such as a physician, medical personnel, etc. The physician may choose to define a minimum and a maximum value associated with the probability value generated by the model, based on which the alert may be generated. The method steps associated with generation of the alert is described in further detail in FIG 4.

FIG 3 illustrates a flowchart of a method 300 of imputing missing values in the medical dataset, according to an embodiment of the invention. At step 301, the medical dataset comprising one or more feature values is received. At step 302, a determination is made to identify the presence of a missing value associated with one or more features in the medical dataset. If a missing value is identified, at step 303, a value which can substitute the missing value is determined. In the embodiment, a value preceding the missing value is determined. In a further embodiment, the process of determining the preceding value associated with the feature may continue until a value is identified in the medical dataset.

At step 304, a check is performed to determine if the substitution value determined meets a pre-determined threshold. In an embodiment, the one or more feature values may be valid for use only if the feature values lie within a time threshold. The time threshold may vary depending on the features in the medical dataset. For example, the values associated with vital signs of the patient are valid only in a time range of 4 to 6 hours. For example, the blood analyte levels associated with the patient are valid only in a time range of 22 to 24 hours. Therefore, if the determined substitution value lies outside of the pre-determined threshold, the imputation is not performed and the value is labelled as missing, at step 305. However, if the substitution value lies within the pre-determined threshold, the missing value in the medical dataset is replaced with the substitution value, at step 306. At step 302, if no missing value is determined in the medical dataset, the one or more feature values are processed further, at step 307, to determine the onset of sepsis in the patient (as described in FIG 2).

FIG 4 illustrates a flowchart of a method 400 of generating an alert indicating the onset of sepsis in the patient, according to an embodiment of the present invention. At step 401, the probability value generated by the trained machine learning model is received by the processing unit 101. At step 402, it is determined if the probability value exceeds a first pre-defined threshold. The first pre-defined threshold is associated with the probability value indicating onset of sepsis in a patient. Therefore, a probability value exceeding the first pre-defined threshold indicates the onset of sepsis in the patient. In an embodiment, the first pre-defined threshold is changeable as per the requirements of the user. Alternatively, the first pre-defined threshold may be changeable based on a condition of the patient. For example, the first pre-defined threshold may be in the range of 0.5 and 1 such that if the probability value generated by the model exceeds 0.5, an alert may be generated. Therefore, at step 404, if the probability value exceeds the first pre-defined threshold, an alert is generated. The alert is a warning indicative of onset of sepsis in the patient. However, if the probability value does not exceed the first pre-de-fined threshold at step 402, no alert is generated at step 403.

At step 405, a total number of alerts generated is identified. This may be performed at regular time intervals such that the number of alerts generated within a defined time period is identified. At step 406, it is determined if the total number of alerts generated within the defined time period exceeds a second pre-defined threshold. The second pre-defined threshold is indicative of maximum number of alerts required to be generated within the defined time period, to generate an alarm indicative of onset of sepsis. If the total number of alerts generated within the defined time period exceed the second pre-defined threshold, at step 407 an alarm is generated indicating the onset of sepsis in the patient. However, if the second pre-defined threshold is not exceeded, no alarm may be generated.

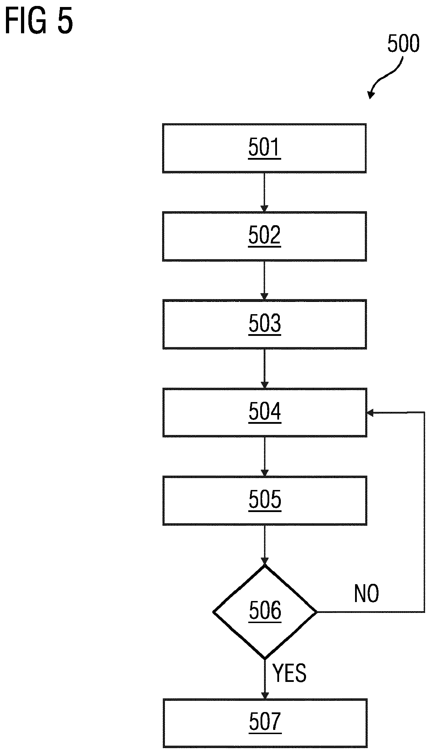

FIG 5 illustrates a flowchart of a method 500 of training a machine learning model to determine an onset of sepsis in a patient, according to an embodiment of the present invention. At step 501, a medical dataset associated with a patient is received. The medical dataset comprises a plurality of features associated with the patient. At step 502, one or more features from the plurality of features in the medical dataset is determined. Such one or more features include parameters associated with the patient which are indicators of onset of sepsis at a given point in time. At step 503, the machine learning model is received by the processing unit 101. At step 504, a probability value is determined by the machine learning model. The machine learning model processes the one or more features from the medical dataset to determine the probability value for onset of sepsis in the patient.

At step 505, sepsis data related to the medical dataset is received. In the embodiment, sepsis data indicates an onset of sepsis or indicates no presence of sepsis at a defined time period in the patient associated with the medical dataset. Sepsis data includes medical dataset which has been labelled for indication of sepsis. Such labelling may be performed by a physician or any expert based on values associated with the one or more features. In an embodiment, the sepsis data may include one or more medical datasets which may be historically recorded for a plurality of patients, for defined time periods. For example, the sepsis data is based on Sepsis-3 criteria and comprises 40 features including 8 vital signs, 26 blood analyte measurements and 6 demographic inputs, each recorded on an hourly basis. At step 506, a comparison is made between the probability value determined by the machine learning model and the sepsis data to determine if a difference exists. If there exists a difference, at step 507 the machine learning model is adjusted based on the sepsis data. In an alternate embodiment, a notification may be generated for the user indicating the presence of a difference between the determined probability value and the sepsis data. Further, an input from the user may be requested on a need to adjust the machine learning model and the model may be adjusted based on the user input.

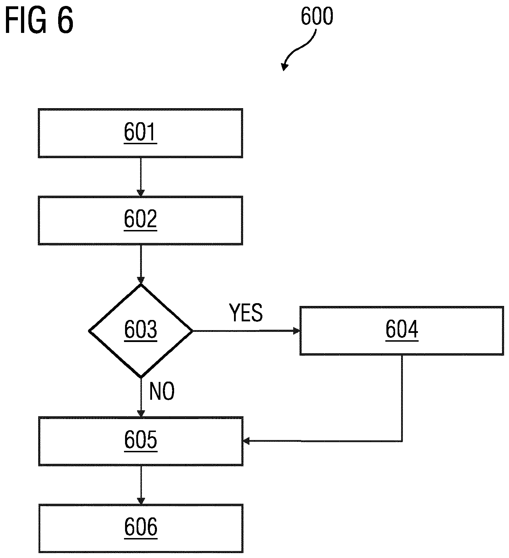

FIG 6 illustrates a flowchart of a method of pre-processing the sepsis data, according to an embodiment of the present invention. At step 601, the sepsis data is received from a source, such as the medical database 112. At step 602, the sepsis data is filtered based on age of the patients to whom the sepsis data belongs and time duration of the patients under intensive care. For example, the filtered sepsis data may include data associated with patients above an age range of 18 to 20 years. Further, the time duration of such patients under intensive care may be more than or equal to 8 hours. In a further embodiment, one or more features indicative of sepsis is extracted from the plurality of features in the sepsis data. For example, a set of 17 features were extracted from the 40 features in the sepsis data along with 4 derivative features (derived from the vital signs and the blood analyte measurements).

At step 603, it is determined if the one or more features have a missing value. If a missing value is identified, at step 604 an imputation of the missing value is performed. The imputation may be based on a feature value preceding or succeeding the missing value in the sepsis data. At step 605, the sepsis data is normalized such that the minimum and maximum thresholds associated with the one or more features is maintained in a range of 1 and 5 respectively. This enables capturing temporal changes in the condition of the patient. Further, at step 606, the labels associated with the sepsis data is modified such that the defined time period indicated by the label is advanced by a range of 5 to 7 hours. This modification of the defined time period enables the machine learning model to identify patterns associated with onset of sepsis in a patient and predict such onset well in advance, i.e. by 5 to 7 hours.

FIG 7 illustrates a working of the machine learning model 700 for determining an onset of sepsis in a patient, according to an embodiment of the present invention. In particular, FIG 7 displays a detailed view of an LSTM network comprising several recurrent neural network blocks RNB.i, RNB.j. Each recurrent neural network block RNB.i, RNB.j uses input data ID.i, ID.j to generate or calculate output data OD.i, OD.j. In an embodiment, the input data ID.i is one or more features from the medical dataset associated with the patient, recorded at time i. Similarly, the input data ID.j is one or more features from the medical dataset associated with the patient, recorded at time j. Output data OD.i and OD.j include probability value generated by the recurrent neural network blocks RNB.i and RNB.j at time I and time j respectively. Additionally, each recurrent neural network block RNB.i, RNB.j takes as additional input intermediate data IBD.i, IBD.j and produces as additional output intermediate data OBD.i, OBD.j, wherein output intermediate data OBD.i, OBD.j can be used as input intermediate data IBD.i, IBD.j within the next step.

It is important to understand that FIG 7 displays an iterative process, which was unfolded for two inputs. In order to adapt for more input data, the iteration can be extended to cover an arbitrary number of input data ID.i, ID.j. Furthermore, the recurrent neural network blocks RNB.i, RNB.j are the same up to a number of internal states IG.i, IG.j, OG.i, OG.j, FG.i, FG.j. In particular, this implies that the output of a neural network block RNB.i, RNB.j only depends on the input data ID.i, ID.j, the additional input intermediate data IBD.i, IBD.j and the internal states IG.i, IG.j, OG.i, OG.j, FG.i, FG.j.

In this embodiment, the neural network is an LSTM network, and the recurrent neural network block RNB.i, RNB.j has internal states denoted as input gate IG.i, IG.j, output gate OG.i, OG.j and forget gate FG.i, FG.j. In particular, the value of these internal states can be calculated as

$$ij = o(W(x,I) * xj + W(y,I) * yi + W(c,I) \cdot ci + b(I))$$

$$fj = o(W(x,F) * xj + W(y,F) * yi + W(c,F) \cdot ci + b(F))$$

$$oj = o(W(x,O) * xj + W(y,O) * yi + W(c,O) \cdot cj + b(O))$$

$$cj = fj \cdot ci + ij \cdot tanh(W(x,C) * xj + W(y,C) * yi + b(C))$$

$$yj = oj \cdot tanh(cj)$$

Within this iteration, the operation "·" is a point-wise multiplication, "*" is a convolution operation, and "σ" denotes the Sigmoid function. The values $ij$, $oj$ and $fj$ correspond to the values of the input gate IG.j, the output gate OG.j and the forget gate FG.j. The values $xj$ and $yj$ correspond to the input data ID.j and the output data OD.j of the respective block. The values $ci$ and $cj$ correspond to the intermediate input intermediate data IBD.i and the output intermediate data OBD.i, OBD.j, and are often denoted as "cell state". The values W and b correspond to weights of the network, which are fixed by training the recurrent neural network.

In an alternative embodiment, one can simplify the update by not letting the cell state influence the updating of the input gate IG.i, IG.j, the output gate OG.i, OG.j and the forget gate FG.i, FG.j:

$$ij = o(W(x,I) * xj + W(y,I) * yi + b(I))$$

$$fj = o(W(x,F) * xj + W(y,F) * yi + b(F))$$

$$oj = o(W(x,O) * xj + W(y,O) * yi + b(O))$$

$$cj = fj \cdot ci + ij \cdot tanh(W(x,C) * xj + W(y,C) * yi + b(C))$$

$$yj = oj \cdot tanh(cj)$$

In another alternative embodiment, the calculation of the cell state can be modified in the following way:

$$cj = fj \cdot ci + (1 \cdot fj) \cdot tanh(W(x,C) * xj + W(y,C) * yi + b(C))$$

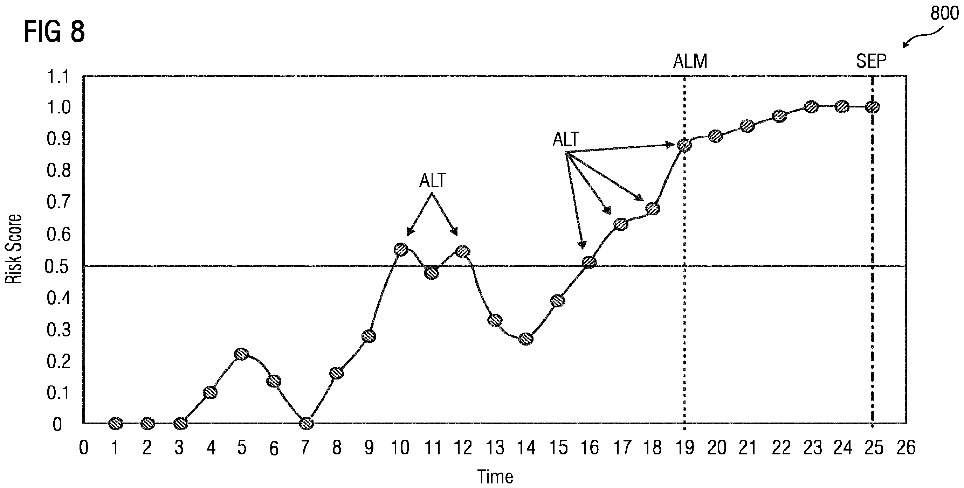

FIG 8 illustrates a graphical representation 800 for monitoring probability value generated by the trained machine learning model based on which alerts and alarms indicating onset of sepsis in a patient is generated, according to an embodiment of the present invention. X-axis of the graphical representation 800, represents time period for which the probability values are generated by the model. In the present embodiment, the time interval defined is one hour. Y-axis of the graphical representation 800 represents probability value indicative of onset of sepsis in the patient. In the embodiment, the first pre-defined threshold is set at a probability value of 0.5. Therefore, if the probability value generated by the trained machine learning model exceed 0.5 an alert ALT is generated. Further, the second pre-defined threshold is set at 4 alerts ALT generated in succession, i.e. an alert ALT generated for every hour for four hours. In the embodiment, the number of alerts ALT generated exceeds the second pre-defined threshold at 19th hour since an alert ALT is generated at every hour starting 16th hour of patient monitoring. Therefore, at the 19th hour, an alarm ALM is generated indicative of onset of sepsis SEP in the patient.

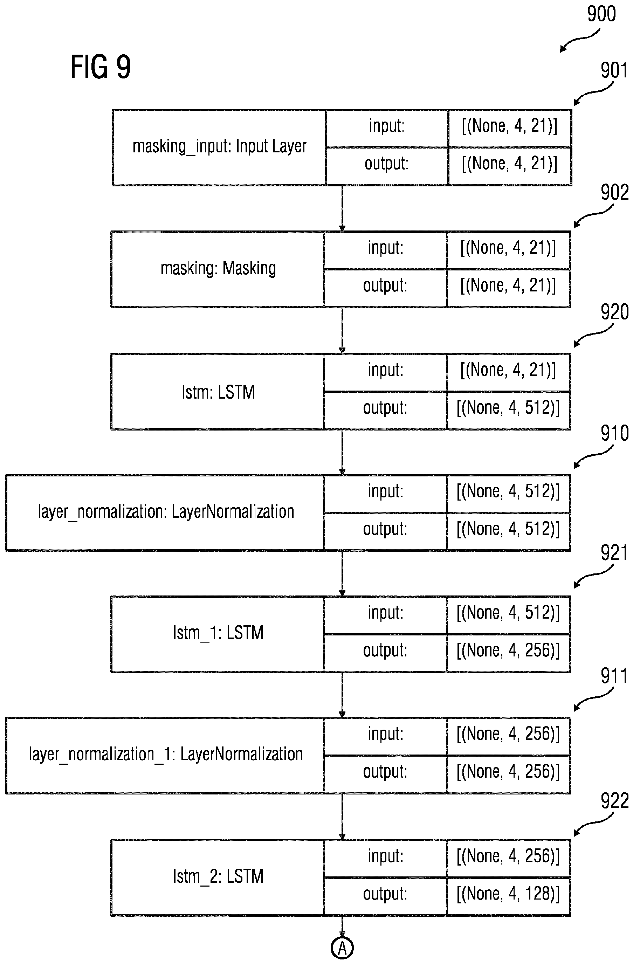

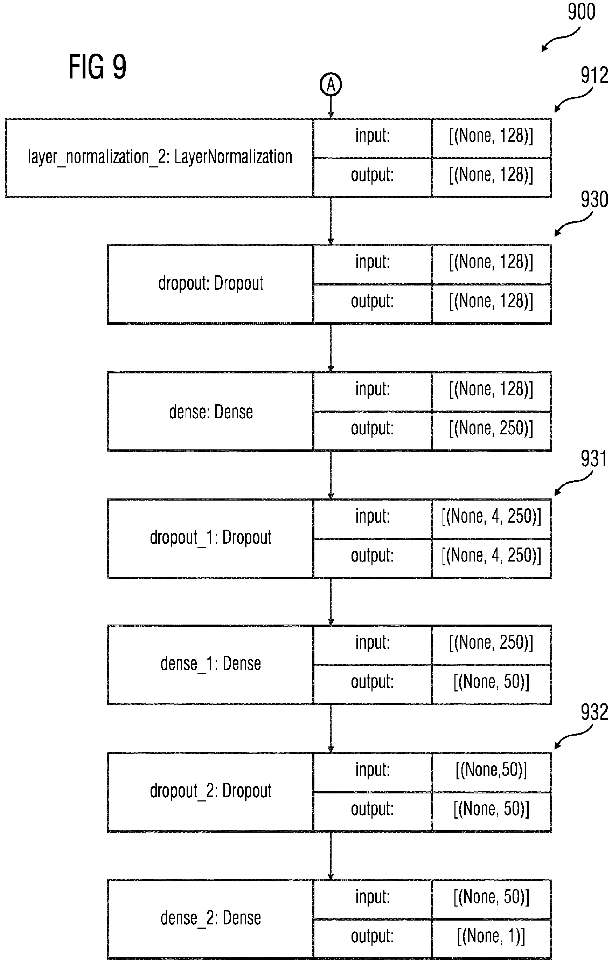

FIG 9 illustrates a further embodiment of the machine learning model. The model architecture includes additional layers, i.e. masking layers 901, 902 and layer normalizations 910, 911, 912, along with LSTM 920, 921, 922. The masking layers 901, 902 disallow missing values in the medical dataset from participating in determining the probability value indicative of onset of sepsis. Layer normalizations 910, 911, 912 stabilize hidden-state dynamics of the recurrent neural network. The model 900 further includes dropout layers 930, 931, 932 and Early Stopping criteria to guard against overfitting. Activations used in the model 900 are ReLu and sigmoid activation for final layer. The model 900 is trained using Adam optimizer with minibatches of 256 datapoints and 1e-4 initial learning rate. The model uses Local Interpretable Model-agnostic Explanations (LIME) and/or SHapley Additive explanations (SHAP) libraries compiled with LSTMs to give real-time feature importance. Advantageously, this enables the user to determine a root cause of the probability value generated by the model.

Evaluation Metrics of the trained machine learning model:

The performance of the model was evaluated with standard parameters such as accuracy, precision, sensitivity and specificity. Additionally, two more parameters were considered for evaluation of model's performance, i.e., utility score and false alarm per true alarm rate. The utility function rewards classifiers for early prediction of sepsis and penalizes the classifiers for late/missed predictions and for predictions of sepsis in non-sepsis patients. The model (for the alarm criteria of 3 warnings in 5 hours) achieves an accuracy of 92.01%, precision of 96.96%, with sensitivity and specificity values of 87.03% and 96.99% respectively. Further, the model achieves a utility score of 0.74 (on a scale of 0 to 1). Additionally, the model achieves a median lookahead time of 5-7 hours. Therefore, advantageously, the model predicts the onset of sepsis 5-7 hours ahead in time. The model achieves false alarm rate of 3% and a false alarm per true alarm rate of 3.45%.

The advantage of the invention is the method and device enable effective determination of onset of sepsis in a patient. The invention determines onset of sepsis in a patient around 5 to 7 hours in advance. Therefore, mortality rates associated with sepsis can be reduced due to timely treatment to the patient. Additionally, the invention allows the user to determine the root cause behind the probability value determined by the model. Therefore, this enables the user to take right steps in correcting/modifying a course of treatment associated with the patient. Furthermore, the model provides the probability value based on up-to-date feature values associated with the patient. Therefore, outdated feature values are eliminated for analysis. This enables higher accuracy in prediction of onset of sepsis in the patient.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

**Claims**

1. A method (200) of determining an onset of sepsis in a patient, the method (200) comprising:

   receiving, by a processing unit (101), at least one medical dataset associated with the patient, wherein the medical dataset comprises a plurality of features;
   extracting, by the processing unit (101), one or more features from the medical dataset, wherein the one or more features comprises parameters associated with the patient which are indicators of sepsis;
   imputing, by the processing unit (101), at least one missing value in the medical dataset, wherein the missing value is associated with the features in the medical dataset;
   determining, by the processing unit (101), an output parameter indicative of the onset of sepsis in the patient by using the one or more features and the at least one imputed missing value in the medical dataset as an input for one or more trained machine learning model (700); and
   generating, by the processing unit (101), an alert (ALT) indicating the onset of sepsis in the patient if the output parameter fulfills a pre-defined criterion associated with sepsis.

2. The method (200) according to claim 1, further comprising normalizing the one or more features from the medical dataset, wherein normalizing comprises defining a uniform minimum and maximum threshold for each of the one or more features.

3. The method (200) according to claim 1 or 2, wherein imputing the at least one missing value in the medical dataset comprises:

   determining, by the processing unit (101), a missing value associated with the features in the medical dataset;
   determining, by the processing unit (101), a value preceding the missing value associated with the features; and
   substituting, by the processing unit (101), the missing value with the value preceding the missing value or the value succeeding the missing value associated with the features in the medical dataset.

4. The method (200) according to any of the aforementioned claims, wherein the one or more features in the medical dataset associated with the patient comprises at least one of vital signs associated with the patient, analytes present in a blood sample of the patient, and derivative parameters associated with the vital signs and the analytes present in a blood sample of the patient.

5. The method (200) according to any of the aforementioned claims, wherein the output parameter indicative of the onset of sepsis in the patient is a probability score, wherein a probability value of 0 indicates no sepsis and a probability value of 1 indicates onset of sepsis.

6. The method (200) according to any of the aforementioned claims, wherein generating the alert indicating the onset of sepsis in the patient comprises:

   determining, by the processing unit (101), if the probability value associated with the patient exceeds a first pre-defined threshold; and
   generating, by the processing unit (101), a warning (ALT) if the probability value exceeds the first pre-defined threshold.

7. The method (200) according to claim 6, further comprising:

   identifying, by the processing unit (101), a number of generated warnings (ALT) indicative of the onset of sepsis;
   determining, by the processing unit (101), if the number of generated warnings (ALT) exceeds a second pre-defined threshold; and
   generating, by the processing unit (101), an alarm (ALM) indicative of the onset of sepsis if the number of generated warnings exceeds the second pre-defined threshold.

8. The method (200) according to any of the aforementioned claims, wherein the trained machine learning model (700) is a recurrent neural network, in particular, comprising a long-short term memory block.

9. A method (500) of training a machine learning model (700) for determining an onset of sepsis in a patient, the method (500) comprising:

   receiving, by a processing unit (101), a medical dataset associated with a patient, wherein the medical dataset comprises a plurality of features associated with the patient;
   extracting, by the processing unit (101), one or more features from the plurality of features in the medical dataset, wherein the one or more features comprises parameters associated with the patient which are indicators of onset of sepsis at a given point in time;
   receiving, by the processing unit (101), the machine learning model (700);
   determining, by the machine learning model (700), a probability value for the onset of sepsis in the patient based on the one or more features present in the medical dataset;
   receiving, by the processing unit (101), sepsis data related to the medical dataset, wherein the sepsis data indicates an onset of sepsis or indicates no presence of sepsis at a defined time period in the patient associated with the medical dataset; and
   adjusting the machine learning model (700) based on an outcome of a comparison between the probability value and the sepsis data.

10. The method (500) according to claim 9, wherein the sepsis data comprises a medical dataset labelled to be indicative of onset of sepsis or no presence of sepsis for a defined time period in a patient.

11. The method (500) according to claim 9 and 10, further comprising pre-processing the sepsis data, wherein pre-processing the sepsis data comprises:

   imputing, by the processing unit (101), at least one missing value in the sepsis data, wherein the missing value is associated with one or more features in the sepsis data; and
   normalizing, by the processing unit (101), the one or more features from the sepsis data, wherein normalizing comprises defining a uniform minimum and maximum threshold for each of the one or more features.

12. The method (500) according to any of the aforementioned claims, wherein the label associated with the sepsis data is modified such that the defined time period indicated by the label is advanced by a range of 2 to 10 hours, preferably 3 to 9 hours, more preferably 6 to 8 hours, and most preferably 5 to 7 hours.

13. A sepsis determination device (100) for determining an onset of sepsis in a patient, the device comprising:

one or more processing units (101);
a medical database (112) coupled to the one or more processing units (101), the medical database (112) comprising a plurality of medical datasets associated with the patient and sepsis data; and
a memory (102) coupled to the one or more processing units (101), the memory comprising a sepsis determination module (110) configured to perform the method steps as claimed in any one of claims 1 to 8, using at least one trained machine learning model (700).

14. A computer program product comprising machine readable instructions, that when executed by one or more processing units (101), cause the one or more processing units (101) to perform method steps according to any of the claims 1 to 12.

15. A computer readable medium on which program code sections of a computer program are saved, the program code sections being loadable into and/or executable in a system (100) to make the system (100) execute the method steps according to any one of the claims 1 to 12 when the program code sections are executed in the system (100).

FIG 1

100

| 107 | ⟷ | 106 | ⟷ | 101 |

103
112

102
110

| 105 | ⟷ | | ⟷ | 104 |

FIG 2

200

201

202

203  YES →  204

NO

205

206

207

FIG 3                                              300

```
                        ┌──────────┐
                        │   301    │
                        └──────────┘
                              │
                              ▼
┌──────────┐    NO        ◇◇◇◇◇        YES      ┌──────────┐
│   307    │◄───────────  ◇  302  ◇  ─────────► │   303    │
└──────────┘              ◇◇◇◇◇                 └──────────┘
                                                      │
                                                      ▼
              ┌──────────┐   NO        ◇◇◇◇◇
              │   306    │◄──────────  ◇  304  ◇
              └──────────┘             ◇◇◇◇◇
                                          │ YES
                                          ▼
                                    ┌──────────┐
                                    │   305    │
                                    └──────────┘
```

FIG 4

400

# FIG 5

500

```
┌─────────────┐
│     501     │
└─────────────┘
       │
       ▼
┌─────────────┐
│     502     │
└─────────────┘
       │
       ▼
┌─────────────┐
│     503     │
└─────────────┘
       │
       ▼
┌─────────────┐
│     504     │◄──────┐
└─────────────┘       │
       │              │
       ▼              │
┌─────────────┐       │
│     505     │       │
└─────────────┘       │
       │              │
       ▼              │
      ╱╲          NO  │
     ╱  ╲─────────────┘
    ╱ 506 ╲
     ╲    ╱
      ╲  ╱
       ╲╱
       │ YES
       ▼
┌─────────────┐
│     507     │
└─────────────┘
```

FIG 6                                    600

```
          ┌──────────────────┐
          │       601        │
          └──────────────────┘
                   │
                   ▼
          ┌──────────────────┐
          │       602        │
          └──────────────────┘
                   │
                   ▼
               ◇                    YES    ┌──────────────────┐
            ◇  603  ◇  ──────────────────▶ │       604        │
               ◇                           └──────────────────┘
                   │ NO                              │
                   ▼                                 │
          ┌──────────────────┐                       │
          │       605        │ ◀─────────────────────┘
          └──────────────────┘
                   │
                   ▼
          ┌──────────────────┐
          │       606        │
          └──────────────────┘
```

FIG 7

700

FIG 8

# FIG 9

900

901

| masking_input: Input Layer | input: | [(None, 4, 21)] |
| | output: | [(None, 4, 21)] |

902

| masking: Masking | input: | [(None, 4, 21)] |
| | output: | [(None, 4, 21)] |

920

| lstm: LSTM | input: | [(None, 4, 21)] |
| | output: | [(None, 4, 512)] |

910

| layer_normalization: LayerNormalization | input: | [(None, 4, 512)] |
| | output: | [(None, 4, 512)] |

921

| lstm_1: LSTM | input: | [(None, 4, 512)] |
| | output: | [(None, 4, 256)] |

911

| layer_normalization_1: LayerNormalization | input: | [(None, 4, 256)] |
| | output: | [(None, 4, 256)] |

922

| lstm_2: LSTM | input: | [(None, 4, 256)] |
| | output: | [(None, 4, 128)] |

Ⓐ

900

FIG 9

912

Ⓐ

| layer_normalization_2: LayerNormalization | input: | [(None, 128)] |
| | output: | [(None, 128)] |

930

| dropout: Dropout | input: | [(None, 128)] |
| | output: | [(None, 128)] |

| dense: Dense | input: | [(None, 128)] |
| | output: | [(None, 250)] |

931

| dropout_1: Dropout | input: | [(None, 4, 250)] |
| | output: | [(None, 4, 250)] |

| dense_1: Dense | input: | [(None, 250)] |
| | output: | [(None, 50)] |

932

| dropout_2: Dropout | input: | [(None,50)] |
| | output: | [(None, 50)] |

| dense_2: Dense | input: | [(None, 50)] |
| | output: | [(None, 1)] |

Europäisches
Patentamt

European
Patent Office

Office européen
des brevets

# EUROPEAN SEARCH REPORT

Application Number

EP 21 17 3588

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/052218 A1 (QUINN ROBERT [US] ET AL) 25 February 2021 (2021-02-25) <br> * page 1, paragraph 0003 - page 5, paragraph 0031 * <br> * page 6, paragraph 0049 - page 11, paragraph 0090 * <br> * page 21, paragraph 0179 - page 27, paragraph 0270; tables 1-7 * <br> * figures 1-15B * <br> ----- | 1-15 | INV. <br> G16H50/20 <br> G16H50/30 <br> A61B5/00 <br><br> ADD. <br> G16H10/40 <br> G16H10/60 <br> A61B5/346 <br> G06N3/08 |
| X | LAURITSEN SIMON MEYER ET AL: "Early detection of sepsis utilizing deep learning on electronic health record event sequences", ARTIFICIAL INTELLIGENCE IN MEDICINE, ELSEVIER, NL, vol. 104, 19 February 2020 (2020-02-19), XP086155697, ISSN: 0933-3657, DOI: 10.1016/J.ARTMED.2020.101820 [retrieved on 2020-02-19] <br> * the whole document, in particular pages the abstract and sections 1, 2.1-2.5 on pages 1-5, and section 4,1 pages 8 and 9 * <br> ----- | 1-15 | |
| A | Anonymous: "Imputation (statistics) - Wikipedia", <br> , <br> 30 April 2021 (2021-04-30), XP055854855, Retrieved from the Internet: URL:https://en.wikipedia.org/w/index.php?title=Imputation_(statistics)&oldid=1020696195 [retrieved on 2021-10-26] <br> * the whole document * <br> ----- | 1-15 | |

TECHNICAL FIELDS
SEARCHED (IPC)

G16H
G06N
A61B

The present search report has been drawn up for all claims

| Place of search | Date of completion of the search | Examiner |
|---|---|---|
| Munich | 26 October 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 089 687 A1**

## ANNEX TO THE EUROPEAN SEARCH REPORT
## ON EUROPEAN PATENT APPLICATION NO.

EP 21 17 3588

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2021

| Patent document cited in search report | Publication date | Patent family member(s) | Publication date |
|---|---|---|---|
| US 2021052218 A1 | 25-02-2021 | NONE | |